# EUROPÄISCHE PATENTANMELDUNG

(11) **EP 3 539 943 A1**
(43) Veröffentlichungstag der Anmeldung: **18.09.2019**
(21) Anmeldenummer: 18162059.2
(22) Anmeldetag: 15.03.2018
(51) Int. Cl.: C07C 241/00, C07C 243/02

(54) **VERFAHREN ZUR HERSTELLUNG VON N-ALKYL-NITRATOETHYLNITRAMINEN**

(71) Anmelder: Nitrochemie Aschau GmbH, 84544 Aschau (DE)
(72) Erfinder: Nieder, Anian, 84453 Mühldorf am Inn (DE); Wünsche, Tobias, 84453 Mühldorf am Inn (DE); Friedrichs, Anne Theresa, 81241 München (DE); Kainz, Johannes, 84453 Mühldorf am Inn (DE); Huber, Alexander, 83109 Großkarolinenfeld (DE)
(74) Vertreter: Lieck, Hans-Peter

(57) **Zusammenfassung**

Die vorliegende Erfindung betrifft ein kontinuierliches Verfahren zur Herstellung von N-Alkyl-nitratoethylnitraminen (NENA Verbindungen).

## Beschreibung

Die vorliegende Erfindung betrifft ein Verfahren zur Herstellung von N-Alkyl-nitratoethylnitraminen (NENA Verbindungen).

Nitratoethylnitramine (NENA Verbindungen) sind als energetische Weichmacher und als Bestandteile von Treibmitteln und Explosivstoffen von großem Interesse. Wichtige Beispiele für Nitratoethylnitramine sind Methyl-NENA, Ethyl-NENA, Butyl-NENA und DINA. Die Herstellung von solchen Verbindungen durch ein kontinuierliches Verfahren ist in EP 1 196 374 und in US 6,262,301 beschrieben. Dieses Verfahren weist jedoch etliche Nachteile auf. Es erfordert beispielsweise einen aufwendigen maschinellen Aufbau (z.B. durch Rührwerk, Wärmetauscher, Kessel, Leitungen usw.), was mit allgemein hohen Sicherheitsanforderungen, einem hohen Wartungs- und Reinigungsaufwand und damit verbundenen längeren Wartungs- und Reinigungsausfällen, einer Immobilität der Anlage und von Anlagenteilen, sowie einem großen Platzbedarf der Gesamtanlage verbunden ist. Das beschriebene Verfahren erfordert außerdem eine aufwändige und komplizierte Temperatur- und Reaktionskontrolle. Ferner ist das An- und Abfahrprozedere des beschriebenen Verfahrens kompliziert und aufwändig. Durch die Entstehung von zündfähigen Dampf/Gasgemischen über dem Flüssigkeitslevel im Reaktorkessel (potentiell hypergolisches Gemisch) ergibt sich ferner ein hohes Zündrisiko. Zudem ist bei dem beschriebenen Verfahren die Vorlage der Reaktanten in den Reaktorkesseln notwendig und es werden weiterhin große Chemikalienmengen eingesetzt, was zu einer großen reaktiven Masse führt. Außerdem ist für das bekannte Verfahren nur die Synthese von Einzelkomponenten und nicht von Produktgemischen beschrieben.

Es war somit die Aufgabe der vorliegenden Erfindung, ein verbessertes Verfahren zur Herstellung von N-Alkyl-nitratoethylnitraminen (NENA Verbindungen) bereitzustellen.

Die vorliegende Erfindung betrifft ein Verfahren zur Herstellung einer Verbindung der Formel (I): 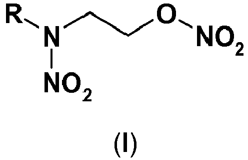 wobei R eine Alkylgruppe mit 1 bis 6 Kohlenstoffatomen oder eine Gruppe der Formel -CH₂-CH₂-O-NO₂ ist, oder von Mischungen von zwei oder mehreren solcher Verbindungen,
welches die Umsetzung einer Verbindung der Formel (II): 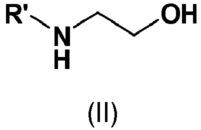 wobei R' eine Alkylgruppe mit 1 bis 6 Kohlenstoffatomen oder eine Gruppe der Formel -CH₂-CH₂-OH ist, oder die Umsetzung von entsprechenden Mischungen von zwei oder mehreren solcher Verbindungen
mit Salpetersäure und anschließend mit einer Mischung aus mindestens einem Säureanhydrid und einem Chlorid-haltigen Katalysator in einem kontinuierlichen Verfahren umfasst, dadurch gekennzeichnet, dass die Umsetzungen bei einem Druck von 0,5 bar über Atmosphärendruck bis zu einem Druck von 1000 bar über Atmosphärendruck durchgeführt werden.

Bei den erfindungsgemäßen Umsetzungen ist ein Druck von 0,5 bar über Atmosphärendruck bis 18 bar über Atmosphärendruck bevorzugt. Des Weiteren bevorzugt ist ein Druck von 1 bar über Atmosphärendruck bis 16 bar über Atmosphärendruck. Weiterhin bevorzugt ist ein Druck von 2 bis 15 bar über Atmosphärendruck. Besonders bevorzugt ist ein Druck von 3 bis 12 bar über Atmosphärendruck; insbesondere von 4 bis 10 bar über Atmosphärendruck. Ein gegenüber Atmosphärendruck erhöhter Druck unterdrückt die Bildung von Gasblasen und damit verbundene Probleme.

Bei dem Verfahren der vorliegenden Erfindung können sowohl symmetrische als auch gemischte Anhydride verwendet werden. Beispiele für geeignete Säureanhydride sind Trifluoressigsäureanhydrid, Essigsäureanhydrid oder Acetylnitrat. Bevorzugt ist Essigsäureanhydrid.

Vorzugsweise wird das Reaktionsgemisch nach den Umsetzungen mit Wasser oder einer wässrigen Lauge in Kontakt gebracht (gequencht).

Bevorzugt ist R eine Methyl-, Ethyl- oder Butylgruppe oder eine Gruppe der Formel -CH₂-CH₂-O-NO₂.

Weiter bevorzugt ist R' eine Methyl-, Ethyl- oder Butylgruppe oder eine Gruppe der Formel -CH₂-CH₂-OH.

Weiterhin bevorzugt wird die Umsetzung mit Salpetersäure bei einer Temperatur von -20°C bis 20°C durchgeführt; insbesondere bei einer Temperatur von -10°C bis 10°C oder bei einer Temperatur von -15°C bis -5°C.

Weiter bevorzugt wird die Umsetzung mit einer Mischung aus mindestens einem Säureanhydrid und einem Chlorid-haltigen Katalysator bei einer Temperatur von 20°C bis 40°C durchgeführt; insbesondere bei einer Temperatur von 27°C bis 36°C oder bei einer Temperatur von 25°C bis 35°C.

Beispiele für Chlorid-haltige Katalysatoren sind Chlorwasserstoff (z.B. als Salzsäure) sowie andere Chlorid-haltige Katalysatoren, wie organische und anorganische Chlorsalze oder Chlorverbindungen, wie z.B. Zink(II)chlorid, Ammoniumchlorid oder Triethylammoniumchlorid, sowie Metallchloride wie beispielsweise Siliciumchloride (z.B. Trimethylsilylchlorid und Tetrachlorsilan) oder Titan(VI)chlorid, oder organische Chlorverbindungen, wie z.B. Acetylchlorid und andere Chlorcarbonsäuren. Diese Metallchloride oder Chlorverbindungen setzten bei Kontakt mit Wasser o.ä. HCl in situ frei. Typischerweise werden 0,02 - 2 mol-% Chlorid im Essigsäureanhydrid für die Reaktion eingemischt.

Bevorzugt wird als Chlorid-haltiger Katalysator Chlorwasserstoff (insbesondere als Salzsäure) eingesetzt. Die Verwendung von Chlorwasserstoff als Chlorid-haltiger Katalysator hat insbesondere gegenüber der Verwendung von Zink(II)chlorid den Vorteil, dass keine Metallsalze bei der Reaktion anfallen, welche den Mikroreaktor verunreinigen oder verstopfen können. Außerdem ist der Einsatz von Chlorwasserstoff billiger und umweltfreundlicher als beispielsweise die Verwendung von Zink(II)chlorid. Die Verwendung von Chlorwasserstoff bietet weiterhin den Vorteil, dass dieser eine bessere Mischbarkeit mit dem übrigen Reaktionsgemisch aufweist und somit keine Vorbereitungszeit für die Mischung benötigt wird. So muss bei dem in EP 1 196 374 beschriebenen Verfahren das Zinkchlorid in Essigsäureanhydrid gelöst werden, was mehrere Stunden in Anspruch nimmt.

Die bei der Umsetzung verwendete Salpetersäure kann in einer Konzentration von 65-100% (in Wasser) eingesetzt werden. Bevorzugt wird Salpetersäure mit einer Konzentration von 75 bis 96% (in Wasser) verwendet. Besonders bevorzugt wird Salpetersäure mit einer Konzentration von 80 bis 90% (in Wasser) verwendet. Die Verwendung von Salpetersäure mit einer Konzentration von 75 bis 96% (insbesondere von 80 bis 90%) hat den Vorteil, dass sich die Reaktion besser kontrollieren lässt, da bei der Verwendung von konzentrierter Salpetersäure die Gefahr einer punktuellen Überhitzung gegeben ist. Es wurde überraschenderweise gefunden, dass das erfindungsgemäße Verfahren nicht nur mit konzentrierter Salpetersäure, sondern auch mit verdünnter Salpetersäure durchgeführt werden kann.

Besonders bevorzugt wird mit dem erfindungsgemäßen Verfahren ein Gemisch von Ethyl-NENA (R = Ethyl) und Methyl-NENA (R = Methyl) hergestellt; insbesondere in einem Gewichtsverhältnis (Ethyl-NENA: Methyl-NENA) von 60:40 bis 30:70; besonders bevorzugt in einem Gewichtsverhältnis von 50:50 bis 40:60. Die Verwendung eines solchen Gemisches bietet den Vorteil, dass durch die Gegenwart von Ethyl-NENA auf ein zusätzliches Lösungsmittel verzichtet werden kann und der Feststoff Methyl-NENA nicht separat aufgereinigt werden muss (insbesondere durch Umkristallisation). Darüber hinaus ist kristallines Methyl-NENA bezüglich des Risikos bei Umgang, Verarbeitung und hinsichtlich der Lagerungs- und Transportrichtlinien als kritischer zu betrachten als in einer Lösung in Ethyl-NENA.

Bevorzugt werden die Umsetzungen der vorliegenden Erfindung in einem oder mehreren Durchflussreaktor(en) durchgeführt.

Als Durchflussreaktor wird vorzugsweise ein Rohrreaktor verwendet.

Besonders bevorzugt werden die Umsetzungen der vorliegenden Erfindung in einem oder mehreren Rohrreaktor(en) durchgeführt.

Das bevorzugte Innenvolumen der eingesetzten Rohrreaktoren beträgt von 0,1 µL bis 10000 L.

Die Verwendung von Durchflussreaktoren (insbesondere von Rohrreaktoren) bietet den Vorteil von kurzen Verweilzeiten bei hohem Durchsatz.

Bevorzugt weist der verwendete Durchflussreaktor oder Rohrreaktor mindestens einen Mischer oder integrierte Strukturen oder Schikanen auf, die als Mischer dienen, (insbesondere einen statischen Mischer) auf.

Insbesondere bevorzugt werden die Umsetzungen der vorliegenden Erfindung in einem oder mehreren Mikroreaktor(en) durchgeführt.

Als Mikroreaktoren werden beispielsweise miniaturisierte Durchflussreaktoren (wie z.B. Rohr-Reaktoren) bezeichnet, deren kleinste Kanaldimensionen z.B. im Bereich von 100 bis 10000 µm liegen. Diese Mikroreaktoren können auf der Basis von Metallen, Silizium, Keramik, Polymeren und Gläsern gefertigt werden. Bekannte Namensreaktionen der präparativen Chemie wie Wittig-Reaktion, Knoevenagel-Kondensation, Michael-Addition, Diels-Alder-Reaktion, Suzuki-Kopplung u.a. wurden in Mikroreaktoren mit überwiegend verbesserten Selektivitäten und Umsätzen erfolgreich durchgeführt. Mikroreaktoren zeichnen sich durch eine sehr genaue und kontinuierliche Prozessführung, einen hohen Wärmeaustausch-Koeffizienten, sehr gute Reaktionskinetik, kurze Verweilzeit der Reaktanden und gute Weiterverarbeitung von instabilen Zwischenprodukten aus (siehe z.B. US 2014/0350274 A1 und Monbaliu et al., Chem. Today 2011, 29, 50).

Durch den Einsatz von Mikroreaktoren ergeben sich etliche Vorteile. Die reaktive Masse und die Risikomengen können minimiert werden. Durch die Umsetzung von kleinen Mengen bei optimaler Temperaturkontrolle (optimales Oberflächen-VolumenVerhältnis und gute Wärmeübertragung z.B. durch einen integrierten Wärmetauscher) ergibt sich eine hervorragende Reaktionskontrolle sowie eine optimale Reaktionsführung (Erhöhung der Ausbeuten und Vermeidung von Nebenprodukten). Ferner entsteht bzw. sammelt sich kein zündfähiges Dampf- oder Gasgemisch an (insbesondere bei einer Reaktion unter Druck); eventuell entstehende Gasblasen von reaktiven Gemischen werden ausgespült. Das erfindungsgemäße Verfahren zeichnet sich außerdem durch ein einfaches An- und Abfahrprozedere mit guter Automatisierungsmöglichkeit ("Plug & Play") aus. Das Verfahren der vorliegenden Erfindung erlaubt die Synthese nach Bedarf, wodurch eine verminderte Lagermenge resultiert (just-in-time-Produktion). Die verwendeten Mikroreaktoren erfordern einen geringeren Wartungs- und Reinigungsaufwand mit folglich geringeren Produktionsausfällen bei kontinuierlicher Produktionsweise als das herkömmliche Verfahren, da keine mechanischen Teile, wie zum Beispiel ein Rührwerk oder Heizspiralen nötig sind. Das Verfahren der vorliegenden Erfindung ist insgesamt Ressourcen-schonend und die sehr kompakte Bauweise der Mikroreaktoren erfordert einen geringen Platzbedarf und führt zu einer leichteren Versetzbarkeit der Anlage (mobile Produktionsanlage).

Die beim Verfahren der vorliegenden Erfindung vorzugsweise verwendeten Mikroreaktoren umfassen vorzugsweise ein oder mehrere Mikroreaktormodul(e), die parallel oder in Serie geschaltet werden können. Diese Mikroreaktormodule sind vorzugsweise als Durchflussreaktoren (continous flow reactor) ausgestaltet. Ein Mikroreaktor kann mehrere gleiche oder auch verschiedene Mikroreaktormodule umfassen. Die Anzahl hintereinander geschalteter Mikroreaktormodule pro Mikroreaktor kann von 1 bis 15 betragen, wobei bevorzugt 2 bis 11 Module pro Mikroreaktor verwendet werden.

Die Mikroreaktormodule können aus einem oder mehreren Materialien wie Glas, Siliziumcarbid, fluorierten Kunststoffen oder korrosionsbeständigen Stählen, (bevorzugt aus Glas und/oder Siliziumcarbid) gefertigt sein.

Der kleinste Durchmesser bzw. die kleinste Abmessung der Fluidkanäle (oder Reaktandenkanäle) der Mikroreaktormodule beträgt vorzugsweise von 100 µm bis 10000 µm ; besonders bevorzugt von 300 µm bis 7000 µm ; insbesondere von 500 µm bis 3000 µm.

Bevorzugt beträgt das gesamte Innenvolumen der Fluidkanäle (oder Reaktandenkanäle) eines Mikroreaktormoduls von 0.5 ml bis 1000 ml; weiter bevorzugt von 5 ml bis 500 ml; besonders bevorzugt von 10 ml bis 400 ml; insbesondere von 30 ml bis 300 ml.

Die Länge der Fluidkanäle (oder Reaktandenkanäle) des gesamten Mikroreaktors wird durch die Verweilzeit, die Flussrate und das Innenvolumen der einzelnen Mikroreaktormodule und die Reaktionsrate sowie die Reaktionstemperatur bestimmt.

Bevorzugt werden Mikroreaktormodule mit Temperiervorrichtung, wie z.B. einem Temperierbad oder Temperierleitungen, eingesetzt. Besonders bevorzugt werden Reaktormodule mit integrierter Temperiervorrichtung verwendet. Solche integrierten Temperiervorrichtungen können z.B. Kapillaren oder Schichten mit einem Temperiermedium sein. Diese Temperiervorrichtungen können über und/oder unter oder um die Durchflusskanäle, in denen die Reaktionen stattfinden, angeordnet sein.

Als Temperiermedium werden bevorzugt Wasser oder Wasser/Alkoholmischungen (z.B. Glykol, Ethanol oder Isopropanol) verwendet.

Die Verweilzeiten in den Mikroreaktormodulen können von einigen Millisekunden bis zu mehreren Minuten betragen. Bevorzugt werden Verweilzeiten von 0,04 min bis 1 min pro Mikroreaktormodul.

Geeignete Mikroreaktormodule sind z.B. die flowing modules der Fa. Corning SAS G0, G1, G2, G3, G4. Bevorzugt sind G0, G1, G3, und G4; besonders bevorzugt G3 und G4. Bevorzugte Mikroreaktormodule sind z.B. in US 8,534,909 beschrieben.

Bei der vorliegenden Erfindung werden bevorzugt mehrere gleiche oder unterschiedliche Mikroreaktoren hintereinander in Serie geschaltet. Besonders bevorzugt werden bei der vorliegenden Erfindung mindestens zwei (insbesondere mindestens drei) Mikroreaktoren in Serie geschaltet.

Es ist ebenfalls möglich, bei der vorliegenden Erfindung einen oder mehrere Mikroreaktor(en) und einen oder mehrere Durchflussreaktoren (wie beispielsweise Rohrreaktoren) in Serie zu schalten.

Bevorzugt wird die erste Reaktion zwischen N-Alkyl-ethanolamin und Salpetersäure in einem Mikroreaktor (umfassend mindestens ein Mikroreaktormodul) als kontinuierliches Verfahren durchgeführt und das Produkt aus diesem Mikroreaktor in einen weiteren Mikroreaktor (umfassend mindestens ein Mikroreaktormodul) in einer kontinuierlichen Art und Weise überführt, wobei in diesem weiteren Mikroreaktor Essigsäureanhydrid und der Chlorid-haltige Katalysator zugegeben werden.

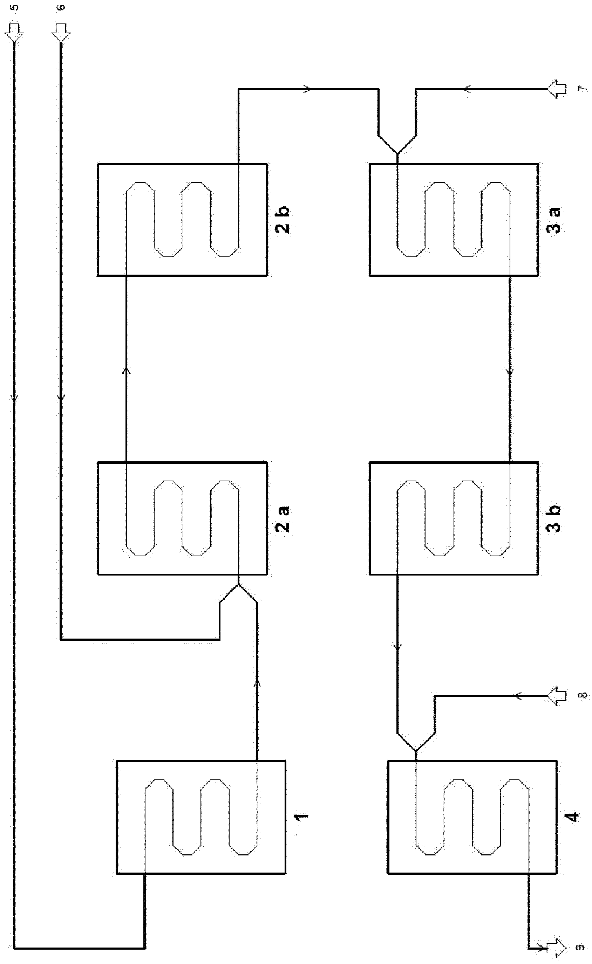

Figur 1 zeigt ein Fließbild einer bevorzugten Vorrichtung zur Durchführung des erfindungsgemäßen Verfahrens.

Im Folgenden wird eine bevorzugte Ausführungsform des Verfahrens der vorliegenden Erfindung im Detail beschrieben. Die Verbindung der Formel (II) bzw. die Mischung davon wird im Folgenden als Amin bezeichnet.

Salpetersäure (65-100%ig in Wasser, bevorzugt 75-96%ig in Wasser, besonders bevorzugt 80-90%ig in Wasser) wird durch einen ersten Mikroreaktor (umfassend mindestens ein Mikroreaktormodul **1**) gepumpt (Flussrate: 10-50 mL/min, Verweilzeiten von 0,1 - 1,1 min, bevorzugt 0,4 - 0,7 min). Der erste Mikroreaktor dient zur Vorkühlung der Säure auf eine Temperatur von -20°C bis 20°C (bevorzugt -10°C bis 10°C).

Die so gekühlte Säure wird in einem zweiten Mikroreaktor (umfassend mindestens ein Mikroreaktormodul **2a** und gegebenenfalls ein oder mehrere Mikroreaktormodul(e) **2b**) mit Amin (Konzentration >50% in Wasser, bevorzugt >96%) bei einem konstanten Amin-Fluss (3 - 20 g/min, bevorzugt 5 - 15 g/min, Verweilzeit 0,1 - 0,7 min, bevorzugt 0,2 - 0,5 min) bei einer Temperatur von -20°C bis 20°C (bevorzugt -10°C bis 10°C) zur Reaktion gebracht.

Die Kühlung der Salpetersäure und die anschließende Reaktion wird bei einem Druck von 0,5 bar über Atmosphärendruck bis zu 18 bar über Atmosphärendruck durchgeführt.

Im zweiten Mikroreaktor kann gegebenenfalls mehr als ein Mikroreaktormodul **2a** angeordnet sein, um die Dosierung von Amin auf mehrere Mikroreaktormodule **2a** zu verteilen. Die Dosierung kann dabei entweder über jeweils unabhängig arbeitende einzelne Dosierpumpen oder über eine Pumpe und die entsprechende Anzahl von Dosierventilen erfolgen.

Das Reaktionsgemisch aus dem zweiten Mikroreaktor wird in einen nächsten Mikroreaktor (umfassend mindestens ein Mikroreaktormodul **3a** und gegebenenfalls ein oder mehrere Mikroreaktormodul(e) **3b**) geleitet und dort mit Essigsäureanhydrid sowie darin gelöstem Katalysator (bevorzugter Katalysatorgehalt für konzentrierte Salzsäure (>25%): 0,5 - 5,0 Vol.-%, bevorzugt 1 - 2 Vol.-%; Flussrate 40 - 120 g/min, bevorzugt 55 - 95 g/min; Verweilzeit 0,1 - 1,1 min, bevorzugt 0,3 - 0,8 min) vermischt und auf 20°C bis 40°C (bevorzugt 27 bis 36°C) erwärmt und zur Reaktion gebracht.

Anschließend kann die Reaktionslösung in einem vierten Mikroreaktor (umfassend mindestens ein Mikroreaktormodul **4**) mit Wasser oder wässriger Lauge (vorzugsweise Natriumhydroxid-, Natriumcarbonat- oder NatriumhydrogencarbonatLösung in Wasser; Konzentration: 5 - 20 Gew.-%) bei 0°C bis 20°C und bei 0 bis 18 bar gequencht und das Rohprodukt gefällt werden. Der Vorteil dieser Variante liegt in der besseren Temperaturkontrolle, sowie in der schnelleren und effizienteren Vermischung bzw. Quenchung.

Alternativ kann das Reaktionsgemisch aus dem dritten Mikroreaktor außerhalb der Fluidkanäle (oder Reaktandenkanäle) bei Normaldruck mit Wasser gequencht und das Rohprodukt ausgefällt werden. Das Rohprodukt fällt dabei vorzugsweise in einer Ausbeute von 60 bis >95 mol-% bezogen auf das Amin und mit einer Reinheit (nach HPLC) von 92 - 100 % an.

Dabei wird das Reaktionsgemisch aus Mikroreaktormodul **3b** in einer Mischkammer mit Wasser bzw. verdünnter Lauge (z.B. Carbonat-Lösung) gequencht bzw. die Reaktion abgefangen. Dabei wird vorzugsweise auf 5 - 20°C temperiert.

Im Unterschied zur ersten Variante mit Mikroreaktormodul **4** lässt sich mittels einer Mischkammer eine größere Menge Quenchmedium zuführen. Ferner kann bei geringerem Druck gearbeitet werden. Dies verbessert die Tröpfchenbildung und erleichtert das Ausgasen von in der Lösung gebundenen Gasen aus der Reaktion oder dem Quenchen, hier insbesondere bei Verwendung einer Carbonat-Lösung.

Die anschließende Trennung kann mittels Zyklon-, Präzipitations-, Membran- oder anderer Phasenseparationsverfahren erfolgen, um die beiden entstehenden Phasen voneinander zu trennen.

Die organische Produktphase aus der Trennung kann mittels Wasser oder einem anderen geeigneten Waschmedium (z.B. verdünnte Lauge oder Salzlösungen) gewaschen und mittels einer zweiten Trennstufe (vgl. erste Trennung) getrennt werden.

Neben der Anpassung der Flussraten kann die Verweilzeit durch modulare Verlängerung der Fluidkanäle (oder Reaktandenkanäle) oder Installierung weiterer Mikroreaktormodule angepasst werden.

### BEISPIELE

### Beispiel 1: Synthese von Ethyl-NENA:

87%ige Salpetersäure mit einer Flussrate von 23 ml/min wird bei einer Verweilzeit von 28 sec auf -6°C gekühlt und in den zweiten Mikroreaktor gefördert. Dort werden bei -6°C 11,5 g/min N-Ethylethanolamin (EEA) zu der vorgekühlten Salpetersäure zudosiert. Nach einer Verweilzeit von 30 sec wird zu diesem Reaktionsgemisch bei einem Fluss von 70 g/min Essigsäureanhydrid mit 1 Vol.-% eingemischter Salzsäure (37%ige Salzsäure in Wasser) zugeleitet. Das Gemisch wird auf 32°C für weitere 34 sec gehalten und mit einem Wasserzustrom von 155 g/min bei 15°C gequencht. Das Rohprodukt fällt mit 92 mol-% Ausbeute (auf das Amin gerechnet) mit einer Reinheit nach HPLC von 98% aus. Der Druck im gesamten Reaktor liegt bei diesen Versuchsbedingungen je nach Messpunkt zwischen 4,5 - 11 bar.

### Beispiel 2: Synthese von Ethyl-/Methyl-NENA (42% Ethyl-NENA, 58% Methyl-NENA, MEN42):

85%ige Salpetersäure mit einer Flussrate von 30 ml/min wird bei einer Verweilzeit von 20 sec auf -6°C gekühlt und in den zweiten Mikroreaktor gefördert. Dort werden bei -6°C 9,5 g/min N-Methylethanolamin/N-Ethylethanolamin (MEA/EEA: 60/40%Gew.) zu der vorgekühlten Salpetersäure dosiert. Nach einer Verweilzeit von 15 sec wird zu diesem Reaktionsgemisch bei einem Fluss von 85 g/min Essigsäureanhydrid mit 1 Gew.-% eingemischtem Zink(II)chlorid zugeleitet. Das Gemisch wird auf 32°C für weitere 29 sec gehalten und mit einem Wasserzustrom von 155 g/min bei 15°C innerhalb von 3 sec gequencht. Das Rohprodukt fällt mit 90 mol-% Ausbeute (auf die Amine gerechnet) mit einer Reinheit nach HPLC von 98% und einem Gewichtsverhältnis von 42% Ethyl-NENA zu 58% Methyl-NENA aus. Der Druck im gesamten Reaktor liegt bei diesen Versuchsbedingungen je nach Messpunkt zwischen 4,5 - 12 bar.

### Beispiel 3: Synthese von Ethyl-/Methyl-NENA (52% Ethyl-NENA, 48% Methyl-NENA, MEN52):

85%ige Salpetersäure mit einer Flussrate von 30 ml/min wird bei einer Verweilzeit von 20 sec auf -6°C gekühlt und in den zweiten Mikroreaktor gefördert. Dort werden bei -6°C 10,0 g/min N-Methylethanolamin/N-Ethylethanolamin (MEA/EEA: 50/50%Gew.) zu der vorgekühlten Salpetersäure dosiert. Nach einer Verweilzeit von 15 sec wird zu diesem Reaktionsgemisch bei einem Fluss von 80 g/min Essigsäureanhydrid mit 1 Vol.-% eingemischter Salzsäure (37%ige Salzsäure in Wasser) zugeleitet. Das Gemisch wird auf 32°C für weitere 30 sec gehalten und mit einem Zustrom von 8%iger Natronlauge 150 g/min bei 20°C innerhalb von 3 sec gequencht. Das Rohprodukt fällt mit 94 mol-% Ausbeute (auf die Amine gerechnet) mit einer Reinheit nach HPLC von 98,5%, sowie einem Gewichtsverhältnis von 52% Ethyl-NENA zu 48% Methyl-NENA aus. Der Druck im gesamten Reaktor liegt bei diesen Versuchsbedingungen je nach Messpunkt zwischen 4,5 - 12,5 bar.

Alternativ kann bei der Verwendung von verdünnter Salpetersäure eine der eigentlichen Synthese vorgeschaltete Verdünnung von hochkonzentrierter Salpetersäure (99-100%ige Salpetersäure) mit Wasser durchgeführt werden. Eine entsprechende Beschreibung ist wie folgt:

### Beispiel 4: Synthese von Ethyl-NENA mit integrierter Säurevormischung:

99%ige Salpetersäure wird im ersten Mikroreaktor mit einem Fluss von 26 mL/min innerhalb von 24 sec auf -6°C gekühlt. Anschließend wird die gekühlte Säure mit 6,0 mL Wasser bei -6°C innerhalb von 18 sec verdünnt. Die so gemischte 87%ige Salpetersäure wird anschließend bei einer Verweilzeit von 19 sec auf -6°C konditioniert und in den zweiten Mikroreaktor gefördert. Dort werden bei -6°C 13 g/min N-Ethylethanolamin (EEA) zu der vorgekühlten verdünnten Salpetersäure dosiert. Nach einer Verweilzeit von 13 sec wird zu diesem Reaktionsgemisch bei einem Fluss von 80 g/min Essigsäureanhydrid mit 1 Vol.-% eingemischter Salzsäure (37%ige Salzsäure in Wasser) zugeleitet. Das Gemisch wird auf 33°C für weitere 29 sec gehalten und mit einem Wasserzustrom von 155 g/min bei 20°C innerhalb von 3 sec gequencht. Das Rohprodukt fällt mit 90 mol-% Ausbeute (auf das Amin gerechnet) mit einer Reinheit nach HPLC von 97% aus. Der Druck im gesamten Reaktor liegt bei diesen Versuchsbedingungen je nach Messpunkt zwischen 4,5 - 12,5 bar.

### Bezugszeichen

- 1: Mikroreaktormodul des ersten Mikroreaktors
- 2a: Mikroreaktormodul des zweiten Mikroreaktors
- 2b: Mikroreaktormodul des zweiten Mikroreaktors
- 3a: Mikroreaktormodul des dritten Mikroreaktors
- 3b: Mikroreaktormodul des dritten Mikroreaktors
- 4: Mikroreaktormodul des vierten Mikroreaktors
- 5: Zufuhr von Salpetersäure
- 6: Zufuhr von Amin
- 7: Zufuhr von Essigsäureanhydrid und Chlorid-haltigem Katalysator
- 8: Zufuhr von Wasser oder wässriger Lauge (Quenchlösung)
- 9: Aufarbeitung/Trennung

## Patentansprüche

1. Verfahren zur Herstellung einer Verbindung der Formel (I): 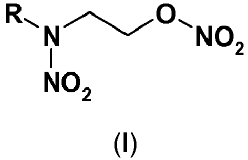 wobei R eine Alkylgruppe mit 1 bis 6 Kohlenstoffatomen oder eine Gruppe der Formel -CH₂-CH₂-O-NO₂ ist, oder von Mischungen von zwei oder mehreren solcher Verbindungen,
welches die Umsetzung einer Verbindung der Formel (II): 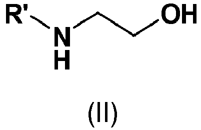 wobei R' eine Alkylgruppe mit 1 bis 6 Kohlenstoffatomen oder eine Gruppe der Formel -CH₂-CH₂-OH ist, oder die Umsetzung von entsprechenden Mischungen von zwei oder mehreren solcher Verbindungen mit Salpetersäure und
anschließend mit einer Mischung aus mindestens einem Säureanhydrid und einem Chlorid-haltigen Katalysator in einem kontinuierlichen Verfahren umfasst,
**dadurch gekennzeichnet, dass** die Umsetzungen bei einem Druck von 0,5 bar über Atmosphärendruck bis zu einem Druck von 1000 bar über Atmosphärendruck durchgeführt werden.

2. Verfahren nach Anspruch 1, **dadurch gekennzeichnet, dass** das Reaktionsgemisch nach den Umsetzungen mit Wasser oder einer wässrigen Lauge in Kontakt gebracht wird.

3. Verfahren nach Anspruch 1 oder 2, **dadurch gekennzeichnet, dass** R eine Methyl-, Ethyl- oder Butylgruppe oder eine Gruppe der Formel -CH₂-CH₂-O-NO₂ ist und R' eine Methyl-, Ethyl- oder Butylgruppe oder eine Gruppe der Formel -CH₂-CH₂-OH ist.

4. Verfahren nach einem der vorhergehenden Ansprüche, **dadurch gekennzeichnet, dass** die Umsetzungen in einem oder mehreren Durchflussreaktor(en) durchgeführt werden.

5. Verfahren nach einem der vorhergehenden Ansprüche 1 bis 3, **dadurch gekennzeichnet, dass** die Umsetzungen in einem oder mehreren Rohrreaktor(en) durchgeführt werden.

6. Verfahren nach einem der vorhergehenden Ansprüche 1 bis 3, **dadurch gekennzeichnet, dass** die Umsetzungen in einem oder mehreren Mikroreaktor(en) durchgeführt werden.

7. Verfahren nach einem der vorhergehenden Ansprüche, **dadurch gekennzeichnet, dass** die Umsetzungen bei einem Druck von 0,5 bar über Atmosphärendruck bis zu einem Druck von 18 bar über Atmosphärendruck, bevorzugt bei einem Druck von 3 bis 12 bar über Atmosphärendruck, durchgeführt werden.

8. Verfahren nach einem der vorhergehenden Ansprüche, **dadurch gekennzeichnet, dass** die Umsetzung mit Salpetersäure bei einer Temperatur von -20°C bis 20°C durchgeführt wird; insbesondere bei einer Temperatur von -10°C bis 10°C oder bei einer Temperatur von -15°C bis -5°C.

9. Verfahren nach einem der vorhergehenden Ansprüche, **dadurch gekennzeichnet, dass** die Umsetzung mit einer Mischung aus mindestens einem Säureanhydrid und einem Chlorid-haltigen Katalysator bei einer Temperatur von 20°C bis 40°C durchgeführt wird; insbesondere bei einer Temperatur von 27°C bis 36°C oder bei einer Temperatur von 25°C bis 35°C.

10. Verfahren nach einem der vorhergehenden Ansprüche, **dadurch gekennzeichnet, dass** Essigsäureanhydrid als Säureanhydrid verwendet wird.

11. Verfahren nach einem der vorhergehenden Ansprüche, **dadurch gekennzeichnet, dass** als Chlorid-haltiger Katalysator Salzsäure verwendet wird.

12. Verfahren nach einem der vorhergehenden Ansprüche, **dadurch gekennzeichnet, dass** die bei der Umsetzung verwendete Salpetersäure mit einer Konzentration von 75 bis 96% in Wasser verwendet wird.

13. Verfahren nach einem der vorhergehenden Ansprüche, **dadurch gekennzeichnet, dass** ein Gemisch von Ethyl-NENA (R = Ethyl) und Methyl-NENA (R = Methyl) hergestellt wird.

14. Verfahren nach einem der vorhergehenden Ansprüche, **dadurch gekennzeichnet, dass** mehrere gleiche oder unterschiedliche Mikroreaktoren hintereinander in Serie geschaltet werden; insbesondere **dadurch gekennzeichnet, dass** mindestens zwei (insbesondere mindestens drei) Mikroreaktoren in Serie geschaltet werden.

15. Verfahren nach einem der vorhergehenden Ansprüche, **dadurch gekennzeichnet, dass** die Reaktion zwischen der Verbindung der Formel (II) und Salpetersäure in einem Mikroreaktor als kontinuierliches Verfahren durchgeführt wird und das Produkt aus diesem Mikroreaktor in einen weiteren Mikroreaktor in einer kontinuierlichen Art und Weise überführt wird, wobei in diesem weiteren Mikroreaktor mindestens ein Säureanhydrid und der Chlorid-haltige Katalysator zugegeben werden.
